# EUROPEAN PATENT APPLICATION

(11) **EP 2 070 549 A1**
(43) Date of publication of application: **17.06.2009**
(21) Application number: 07792413.2
(22) Date of filing: 10.08.2007
(51) Int. Cl.: A61K 45/00, A61K 38/00, A61K 38/22, A61P 25/00, A61P 43/00

(54) **THERAPEUTIC AGENT FOR ACCELERATION OF SPINAL NERVE REPAIR COMPRISING DESACYL GHRELIN OR DERIVATIVE THEREOF AS ACTIVE INGREDIENT**

(30) Priority: 11.08.2006 JP 2006220695
(71) Applicant: University of Miyazaki, Miyazaki-shi Miyazaki 889-2192 (JP); Asubio Pharma Co., Ltd., Minato-ku Tokyo 107-8541 (JP); Japan as represented by president of National Cardiovascular Center, Suita-shi, Osaka 565-8565 (JP)
(72) Inventor: MURAKAMI, Noboru, Miyazaki-shi Miyazaki 880-0033 (JP); NAKAHARA, Keiko, Miyazaki-gun Miyazaki 889-1601 (JP); HASHIMOTO, Miho, Maebashi-shi Gunma 371-0037 (JP); HAYASHI, Yujiro, Ohra-gun Gunma 370-0503 (JP); KANGAWA, Kenji, Suita-shi Osaka 565-8565 (JP)
(74) Representative: Vossius & Partner
(86) International application number: PCT/JP2007/065769
(87) International publication number: WO 2008/018597

(57) **Abstract**

The invention provides a spinal neuron damage treating agent for use in the treatment of spinal neuron damage, or an agent for promoting the proliferation of spinal neuronal cells in the culture of spinal neuronal cells, or an agent for promoting the regeneration of spinal nerves after transplantation of cultured spinal neuronal cells, and the like.

The invention provides an agent that contains a substance that acts on a des-acyl ghrelin specific binding site or a pharmaceutically acceptable salt thereof, the agent being a spinal neuron damage treating agent for use in the treatment of spinal neuron damage, or an agent for promoting the proliferation of cultured spinal neuronal cells in the culture of spinal neuronal cells, or an agent for promoting the regeneration of spinal nerves after transplantation of cultured spinal neuronal cells, and the like.

## Description

### TECHNICAL FIELD

The present invention relates to the spinal neuron proliferating action of substances that act on des-acyl ghrelin specific binding sites. More particularly, the invention relates to agents that contain those substances as an active ingredient, such as spinal nerve damage treating agents for use in the treatment of spinal nerve damage, agents for promoting the proliferation of spinal neurons in the culture of spinal neurons, and agents for promoting the regeneration of spinal nerves after transplantation of cultured spinal neurons.

### BACKGROUND ART

More than 6000 people suffer from spinal cord injury annually as the result of traffic accidents, sport accidents and occupational accidents, with a total of about 110,000 victims being counted across the nation. This is estimated to cost a social loss of as much as 300 billion yen per year and it has been said that there is no method of treatment (Non-Patent Document 1). Spinal nerves have so far been considered to be non-regenerable or non-transplantable; however, as a tissue, the transplantation of spinal nerves is recently being investigated at an experimental level as in the case of fetal spinal cord cells and there is suggested possibility for their treatment by transplantation. Furthermore, in view of the future research on transplantation and regeneration, spinal nerves may be considered to be a tissue the development and regeneration processes of which are anticipated to be verified in the years to come by means of such studies as the research on the development of spinal nerves and regeneration research using cultured fetal spinal neurons. In addition, the research on the transplantation and regeneration of spinal nerves is anticipated to play an important role in spinal cord injury, spinal cord tumor, brain tumor or cranial nerve disorder.

As a matter of fact, the preclinical research on nerve regeneration is making steady advances. Cells and substances that have the potential to repair and regenerate nerves include human stem cells, nasal mucosal cells, myeloid series of cells such as stromal marrow cells, umbilical blood, macrophages, 4-aminopyridine (which is under large-scale phase III clinical trial), etc. Thus, a series of research reports have been made in the last two or three years about new cells and substances that show the potential for nerve repair and regeneration. Some of these studies are in the process of transition from the level of animal experiment to the clinical study in humans and in order to ensure that they will continue to develop constantly, the establishment of a medical center for spinal cord regeneration that is equipped with an integrated research system combining the preclinical with the clinical research is becoming an urgent goal in Japan (Non-Patent Document 2).

Transplantation of spinal cord cells is currently studied with animals at an experimental level and clinical research has begun in order to apply it to the treatment of spinal cord injuries in humans. Cells that are used in preclinical or clinical research include human stem cells, nasal mucosal cells, myeloid series of cells, umbilical blood, macrophages, and tissues from embryos (Non-Patent Document 2).

Another source that is used in the research on nerve regeneration is tissues from embryos. In animal experiments, they drew attention as a source that would show high capability for regeneration and the nerve stem cells extracted from a monkey fetal spinal cord were transplanted in a monkey with a damaged spinal cord, which successfully recovered its function (Non-Patent Document 3); however, fetal cells generally proliferate so slowly that development of a proliferation promoting technique is desired.

Further, the clinical application of GM-CSF, lectin and the like that are used in neuronal regenerative therapy involves concern about cell differentiation at other sites in the human body and side-effects and, hence, it is desired to discover and develop substances that are highly safe while having the intended activity (Non-Patent Document 4).

Des-acyl ghrelin is a peptide identical to ghrelin, a hormone discovered from the stomach in 1999, except that a fatty acid has been eliminated (Non-Patent Document 5 and Patent Document 1).

Ghrelin was first isolated and purified from the rat as an endogenous GHS for the growth hormone secretagogue-receptor 1a (GHS-R1a) (Non-Patent Document 6) and vertebral animals other than rat, such as human, mouse, porcine, chicken, eel, bovine, equine, ovine, frog, trout, and canine, are known to have amino acid sequences of ghrelin having similar primary structures (Patent Document 1).
Human
: GSS(n-octanoyl)FLSPEHQRVQQRKESKKPPAKLQPR (corresponding to SEQ ID NO: 1)
: GSS(n-octanoyl)FLSPEHQRVQRKESKKPPAKLQPR (corresponding to SEQ ID NO:2)
Rat
: GSS(n-octanoyl)FLSPEHQKAQQRKESKKPPAKLQPR (corresponding to SEQ ID NO:3)
: GSS(n-octanoyl)FLSPEHQKAQRKESKKPPAKLQPR (corresponding to SEQ ID NO:4) Mouse
: GSS(n-octanoyl)FLSPEHQKAQQRKESKKPPAKLQPR (corresponding to SEQ ID NO:5) Porcine
: GSS(n-octanoyl)FLSPEHQKVQQRKESKKPAAKLKPR (corresponding to SEQ ID NO:6) Bovine
: GSS(n-octanoyl)FLSPEHQKLQRKEAKKPSGRLKPR (corresponding to SEQ ID NO:7) Ovine
: GSS(n-octanoyl)FLSPEHQKLQRKEPKKPSGRLKPR (corresponding to SEQ ID NO:8) Canine
: GSS(n-octanoyl)FLSPEHQKLQQRKESKKPPAKLQPR (corresponding to SEQ ID NO:9) Eel
: GSS(n-octanoyl)FLSPSQRPQGKDKKPPRV-NH₂ (corresponding to SEQ ID NO:10)
Trout
: GSS(n-octanoyl)FLSPSQKPQVRQGKGKPPRV-NH₂ (corresponding to SEQ ID NO:11)
: GSS(n-octanoyl)FLSPSQKPQGKGKPPRV-NH₂ (corresponding to SEQ ID NO:12) Chicken
: GSS(n-octanoyl)FLSPTYKNIQQQKGTRKPTAR (corresponding to SEQ ID NO:13)
: GSS(n-octanoyl)FLSPTYKNIQQQKDTRKPTAR (corresponding to SEQ ID NO:14)
: GSS(n-octanoyl)FLSPTYKNIQQQKDTRKPTARLH (corresponding to SEQ ID NO:15)
Bullfrog
: GLT(n-octanoyl)FLSPADMQKIAERQSQNKLRHGNM (corresponding to SEQ ID NO:16)
: GLT(n-decanoyl)FLSPADMQKIAERQSQNKLRHGNM (corresponding to SEQ ID NO:16)
: GLT(n-octanoyl)FLSPADMQKIAERQSQNKLRHGNMN (corresponding to SEQ ID NO:17)
Tilapia
: GSS(n-octanoyl)FLSPSQKPQNKVKSSRI-NH₂ (corresponding to SEQ ID NO:18)
Catfish
: GSS(n-octanoyl)FLSPTQKPQNRGDRKPPRV-NH₂ (corresponding to SEQ ID NO:19)
: GSS(n-octanoyl)FLSPTQKPQNRGDRKPPRVG (corresponding to SEQ ID NO:20)
Equine
: GSS(n-butanoyl)FLSPEHHKVQHRKESKKPPAKLKPRb(corresponding to SEQ ID NO:21)

### (In the above designations, amino acid residues are represented by the single-letter code.)

The above-mentioned peptides are those having such a unique structure that the side-chain hydroxyl group in the serine residue (S) or threonine residue (T) at position 3 is acylated with a fatty acid such as octanoic acid or decanoic acid and no physiologically active peptides other than ghrelin that have such a hydrophobic, modified structure have been isolated from the living body. These peptides, if the fatty acid in the serine or threonine residue at position 3 is eliminated, may have the sequence represented by any one of SEQ ID NO:1 to SEQ ID NO:21 and the resulting peptide is called des-acyl ghrelin.

Des-acyl ghrelin has weak affinity for GHS-R1a and its activity for stimulating the secretion of growth hormone (GH) from the pituitary is weak. Recent studies have revealed that ghrelin enhances appetite and, upon subcutaneous administration, increases body weight and body fat (Non-Patent Documents 7 to 9), as well as having such actions as an improvement in cardiac function (Non-Patent Documents 10 to 12). It has been reported that des-acyl ghrelin as well as ghrelin functions to protect the heart muscle by means of inhibiting apoptosis of cardiomyocytes (Non-Patent Document 13), with the suggestion that it participates in the fate of cells as in cell proliferation and cell death. It has also been reported that des-acyl ghrelin shows an anti-proliferation action on prostate cancer cells (Non-Patent Document 14) and des-acyl ghrelin is speculated to act on receptors other than GHS-R1a. As regards the effects that des-acyl ghrelin may exert on the eating behavior, both an enhancing and a suppressing effect have been reported, and there is a report showing that overexpression of des-acyl ghrelin resulted in a smaller physique and lower IGF-1 levels (Non-Patent Document 15).

The present inventors found that ghrelin and des-acyl ghrelin were present in the amniotic fluid of a pregnant mother animal and as the result of a study they made considering the functions and roles of those substances, they found that GHS-R1a was present on the skin cells of embryos and that des-acyl ghrelin had the action of proliferating the fetal skin cells (Non-Patent Document 16). Other actions of des-acyl ghrelin that have been suggested include the effects they may exert on the proliferation of cancer cells (Non-Patent Document 17) but no action on the nerve cell system is known.

To the best knowledge of the present inventors, no report has yet been found that is directed to showing that des-acyl ghrelin specific binding sites are expressed in spinal neurons and that the des-acyl ghrelin acts on spinal neurons to exhibit an action for proliferating such spinal neurons.
[Patent Document 1] WO 01/07475 A1
[Patent Document 2] JP 2005-239712 A
[Non-Patent Document 1] Iryo In Focus (Medicine in Focus), Part II, Saisei Iryo (Regenerative Medicine), 6 "Sekizui Shinkei" Saisei (Regeneration of Spinal Cord Nerves), May 21, 2005 (URL:http://www.ubenippo.co.jp/infocus/saisei/saisei 6.html)
[Non-Patent Document 2] Shinkeisaiseikenkyu ni okeru taijisoshikiriyo ni kannsuru kenkai (Opinion Concerning the Utilization of Fetal Tissues in the Research on Nerve Regeneration), April 5, 2005, Japan Spinal Cord Foundation, an incorporated non-profit organization (URL : http://www.jscf.org/jscf/SIRYOU/igaku/-1/saiboisyoku/jscf050405.html)
[Non-Patent Document 3] Shinkeikansaibo de kotsuzuisonshokaifuku (Recovery from Spinal Cord Injury Using Neural Stem Cells), Article from December 10, 2001 issue of Tokyo Shinbun (URL : http://www.normanet.ne.jp/∼JSCF/SIRYOU/tokyo-2.htm) [Non-Patent Document 4] Shinkeisaiseichiryo (Regeneration of Central Nerves System) (URL : http://www.ins-gbs.co.jp/nerve.html)
[Non-Patent Document 5] Kojima et al. : Nature, 402, pp. 656-660 (1999)
[Non-Patent Document 6] Howard et al. : Science, 273, pp. 974-977 (1996)
[Non-Patent Document 7] Wren et al. : Endocrinology, 141, pp. 4325-4328 (2000)
[Non-Patent Document 8] Nakazato et al.: Nature, 409, 194-198 (2001)
[Non-Patent Document 9] Shintani et al. : Diabetes, 50, pp. 227-232 (2001)
[Non-Patent Document 10] Nakazato et al. : Nature, 409, pp. 194-198 (2001)
[Non-Patent Document 11] Lely et al.: Endocr. Rev., 25, pp. 656-660 (2004)
[Non-Patent Document 12] Korbonits et al. : Front Neuroendocrinol., 25, pp. 27-68 (2004)
[Non-Patent Document 13] Baldanzi et al. : J. Cell Biol., 159, pp. 1029-1037 (2002)
[Non-Patent Document 14] Cassoni et al. : Eur. J. Endocrinol., 150, pp. 173-184 (2004)
[Non-Patent Document 15] Ariyasu et al. : Endocrinology, 146, pp. 355-364 (2005)
[Non-Patent Document 16] Nakahara et al. : Endocrinology, 147, pp. 1333-1342 (2006)
[Non-Patent Document 17] Zhang et al. : J Physiol., 559, pp. 729-737 (2004)

### DISCLOSURE OF THE INVENTION

### PROBLEMS TO BE SOLVED BY THE INVENTION

The present invention relates to providing agents that employ substances having an action for proliferating spinal neurons, such as spinal neuron damage treating agents for use in the treatment of spinal neuron damage, agents for promoting the proliferation of spinal neurons in the culture of spinal neurons, and agents for promoting the regeneration of spinal nerves after transplantation of cultured spinal neurons.

### MEANS FOR SOLVING THE PROBLEMS

As described above, the present inventors found that the growth hormone secretagogue-receptor (GHS-R1a) was present on the skin cells of rat embryos and that des-acyl ghrelin had the action of proliferating the fetal skin cells.

Further, with a view to studying the action of the substances acting on the GHS-R1a in rat embryos and des-acyl ghrelin specific binding sites (receptors), the present inventors searched for the site of presence of GHS-R1a by the RT-PCR technique and found that GHS-R1a was present in spinal neurons and that des-acyl ghrelin specific binding sites were present in such spinal neurons. The present inventors then found that the substances acting on the des-acyl ghrelin specific binding sites would act on spinal neurons to exhibit an action for proliferating such spinal neurons. Furthermore, in a test with rat models suffering a spinal cord injury, des-acyl ghrelin was locally administered in combination with transplantation of cultured spinal neuronal cells, whereupon the inventors confirmed a recovery of the impaired lower limb's function from the spinal cord injury.

Thus, the present invention relates to an agent that contains a substance that acts on a des-acyl ghrelin specific binding site as an active ingredient, the agent being a spinal neuron damage treating agent for use in the treatment of spinal neuron damage, or an agent for promoting the proliferation of cultured spinal neuronal cells, or an agent for promoting the regeneration of spinal nerves after transplantation of cultured spinal neuronal cells.

The present invention also relates to a method that comprises administering a substance that acts on a des-acyl ghrelin specific binding site, the method being a method for treating spinal neuron damage in an individual, or a method for promoting the proliferation of cultured spinal neuronal cells, or a method for promoting the regeneration of spinal nerves after transplantation of cultured spinal neuronal cells in an individual.

Further, the present invention relates to the use of a substance that acts on a des-acyl ghrelin specific binding site for producing a spinal neuron damage treating agent for use in the treatment of spinal neuron damage, or an agent for promoting the proliferation of cultured spinal neuronal cells, or an agent for promoting the regeneration of spinal nerves after transplantation of cultured spinal neuronal cells.

In view of the foregoing, the present invention specifically relates to the following items:
[1] A spinal neuron damage treating agent containing a substance that acts on a des-acyl ghrelin specific binding site or a pharmaceutically acceptable salt thereof as an active ingredient.
[2] The treating agent of [1] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[3] The treating agent of [2] above, wherein the substance is a peptide having the amino acid sequence represented by SEQ ID NO:1.
[4] The treating agent as recited in [1] to [3] above, which contains 0.001 mg to 100 mg of the substance or pharmaceutically acceptable salt thereof.
[5] An agent for promoting the proliferation of cultured spinal neuronal cells that contains a substance that acts on a des-acyl ghrelin specific binding site or a salt thereof as an active ingredient.
[6] The promoting agent of [5] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid
      sequence represented by any one of SEQ ID NO:1 to
      SEQ ID NO:21 and having a spinal neuronal cells
      proliferating action,
      or a derivative thereof.
[7] The promoting agent of [6] above, wherein the substance is a peptide having the amino acid sequence represented by SEQ ID NO:1.
[8] The promoting agent as recited in [5] to [7] above, wherein the content of the substance or salt thereof in the culture medium for spinal neuronal cells is from 0.0000001 mg/L to 0.1 mg/L.
[9] An agent for promoting the regeneration of spinal nerves after transplantation of cultured spinal neuronal cells that contains a substance that acts on a des-acyl ghrelin specific binding site or a pharmaceutically acceptable salt thereof as an active ingredient.
[10] The treating agent of [9] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[11] The treating agent of [10] above, wherein the substance is a peptide having the amino acid sequence represented by SEQ ID NO:1.
[12] The treating agent as recited in [9] to [11] above, which contains 0.001 mg to 100 mg of the substance or pharmaceutically acceptable salt thereof.
[13] A method for treating spinal neuron damage that comprises administering an individual with a substance that acts on a des-acyl ghrelin specific binding site or a pharmaceutically acceptable salt thereof.
[14] The method of [13] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[15] The method of [14] above, wherein the substance is a peptide is a peptide having the amino acid sequence represented by SEQ ID NO:1.
[16] The method as recited in [13] to [15] above, which comprises administering 0.001 mg to 100 mg of the substance or pharmaceutically acceptable salt thereof.
[17] A method for promoting the proliferation of cultured spinal neuronal cells, characterized by using a substance that acts on a des-acyl ghrelin specific binding site or a salt thereof.
[18] The method of [17] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[19] The method of [18] above, wherein the substance is a peptide having the amino acid sequence represented by SEQ ID NO:1.
[20] The method as recited in [17] to [19] above, wherein the content of the substance or salt thereof in the culture medium for the cultured spinal neuronal cells is from 0.0000001 mg/L to 0.1 mg/L.
[21] A method for promoting the regeneration of spinal nerves after transplantation of cultured spinal neuronal cells, which comprises administering an individual with a substance that acts on a des-acyl ghrelin specific binding site or a pharmaceutically acceptable salt thereof.
[22] The method of [21] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[23] The method of [22] above, wherein the substance is a peptide is a peptide having the amino acid sequence represented by SEQ ID NO:1.
[24] The method as recited in [21] to [24] above, which comprises administering 0.001 mg to 100 mg of the substance or pharmaceutically acceptable salt thereof.
[25] Use of a substance that acts on a des-acyl ghrelin specific binding site or a pharmaceutically acceptable salt thereof for producing an agent for treating spinal neuron damage.
[26] The use of [25] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[27] The use of [26] above, wherein the substance is a peptide having the amino acid sequence represented by SEQ ID NO:1.
[28] The use as recited in [25] to [27] above, wherein the agent for treating spinal neuron damage contains 0.001 mg to 100 mg of the substance or pharmaceutically acceptable salt thereof.
[29] Use of a substance that acts on a des-acyl ghrelin specific binding site or a pharmaceutically acceptable salt thereof for producing an agent for promoting the regeneration of spinal nerves after transplantation of cultured spinal neuronal cells.
[30] The use of [29] above, wherein the substance is a peptide selected from the group consisting of:
   (1) a peptide having the amino acid sequence represented by any one of SEQ ID NO:1 to SEQ ID NO:21;
   (2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
   (3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.
[31] The use of [30] above, wherein the substance is a peptide having the amino acid sequence represented by SEQ ID NO:1.
[32] The use as recited in [29] to [31] above, wherein the agent for promoting the regeneration of spinal nerves contains 0.001 mg to 100 mg of the substance or pharmaceutically acceptable salt thereof.

### ADVANTAGES OF THE INVENTION

It has been revealed by the present invention that the substances acting on a des-acyl ghrelin specific binding site have the action of proliferating spinal neuronal cells. It is on the basis of this action that the substances acting on a des-acyl ghrelin specific binding site can be administered to an individual with damaged spinal nerves to successfully treat the spinal nerve damage.

In addition, when spinal neuronal cells are cultured, the substances acting on a des-acyl ghrelin specific binding site may be added to promote cell proliferation, whereby it becomes possible to expedite the use of the cultured spinal neuronal cells in therapy. In other words, in the case of culturing spinal neuronal cells and grafting them to the damaged site of spinal nerves to thereby repair and treat the spinal nerves, it becomes possible to ensure that the cultured spinal neuronal cells are supplied with greater rapidity to the individual.

Further, the cultured spinal neuronal cells may be directly grafted to the affected area and the substances acting on a des-acyl ghrelin specific binding site are then administered to the grafted area, whereby it becomes possible to realize promoted curing.

### BRIEF DESCRIPTION OF THE DRAWINGS

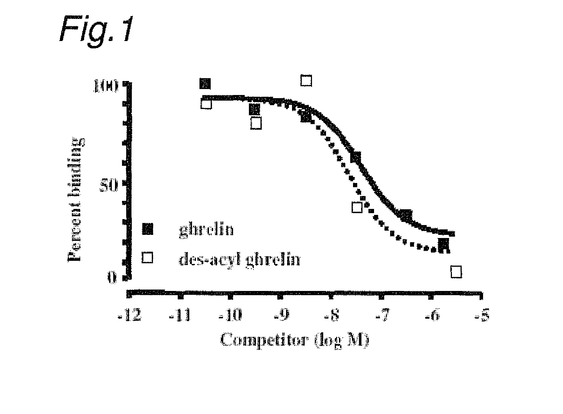

[FIG. 1] FIG. 1 shows the presence of des-acyl ghrelin specific binding sites in membrane fractions of spinal cord.

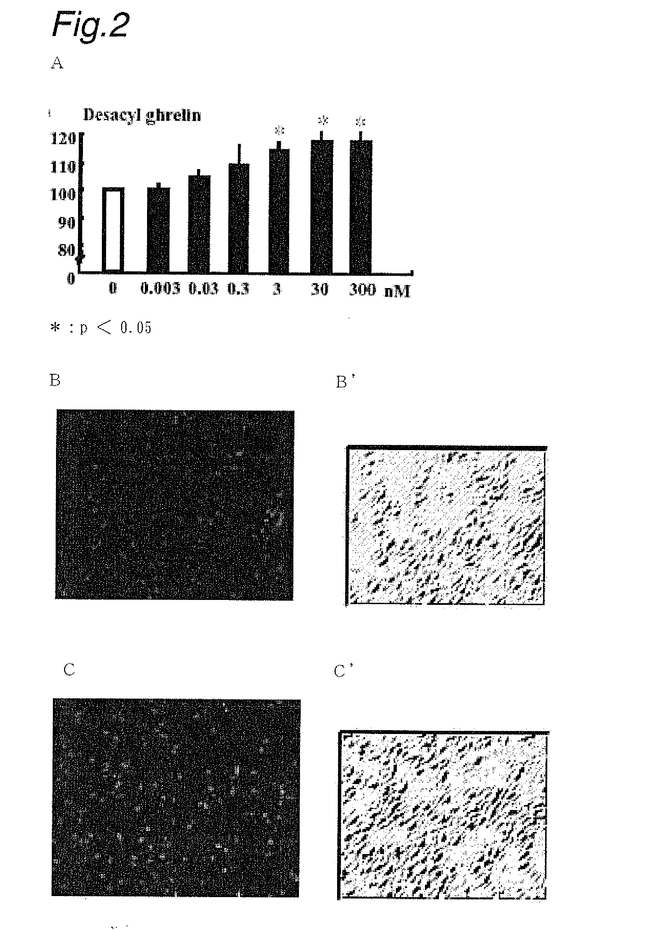

[FIG. 2] FIG. 2 shows the action of des-acyl ghrelin for promoting the incorporation of BrdU into spinal neurons, as well as its cell proliferating action; FIG. 2A shows the action of des-acyl ghrelin for promoting the incorporation of BrdU into spinal neurons and the numerals represent the mean ± SEM (*: p<0.05); FIG. 2B shows BrdU positive cells (immunostained) for the case where physiological saline was allowed to act on spinal neurons; FIG. 2C shows BrdU positive cells (immunostained) for the case where des-acyl ghrelin was allowed to act on spinal neurons; note that FIGS. 2B' and 2C' are diagrams drawn on the basis of FIGS. 2B and 2C.

### BEST MODES FOR CARRYING OUT THE INVENTION

The pharmaceuticals of the present invention can be used as pharmaceuticals for animals (individuals). The substances that can be used in the present invention include substances (ligands) that act on des-acyl ghrelin specific binding sites, as exemplified by des-acyl ghrelin and its derivatives.

The ""peptides" are compounds in which a number of amino acids are connected together by a peptide bond. Here, the amino acids (which may alternatively be designated as amino acid residues) include not only natural amino acids of the general formula: NH₂-CH(R')-COOH where R' has a naturally occurring substituent, but also their D,L-optical isomers and the like. Some natural amino acids may be replaced by modified amino acids (which may alternatively be designated as modified amino acid residues). The modified amino acids include not only those amino acids of the general formula indicated above in which the substituent R' is further modified and their D,L-otpical isomers but also such nonnatural amino acids that the substituent R' in the general formula indicated above has a variety of substituents bonded thereto with or without an intervening moiety such as an ester, ether, thioester, thioether, amide, carbamide or thiocarbamide. Also included are nonnatural amino acids having the amino group in the amino acid replaced by a lower alkyl group.

Hereinafter, the "peptide derivatives" may include such compounds that at least one amino acid in a peptide is replaced by a non-amino acid compound, such compounds that the amino terminus and/or carboxyl terminus of a peptide is modified (say, compounds with the carboxyl terminus being amidated), and such compounds that at least one amino acid in a peptide is replaced by a non-amino acid compound and, in addition, the amino terminus and/or carboxyl terminus is modified.

Hereinafter, the "amino acids" include all kinds of amino acids, as exemplified by L-amino acids, D-amino acids, α-amino acids, β-amino acids, γ-amino acids, natural amino acids, and synthetic amino acids.

Hereinafter, the "modified amino acids" means the above-mentioned amino acids in which any desired group is chemically modified. Particularly preferred are such modified amino acids that the α-carbon in an α-amino acid is chemically modified.

An exemplary des-acyl ghrelin is a peptide having the sequence represented by any one of SEQ ID NO:1 to SEQ ID NO:21. To be more specific, as noted above, des-acyl ghrelin that can be used encompasses not only the des-acyl ghrelin derived from humans but also the des-acyl ghrelin derived from rat, mouse, porcine, bovine and other animals. For a given individual, des-acyl ghrelin derived from the species to which the individual belongs is desirably used; for instance, human derived des-acyl ghrelin is desirably used in humans. The human derived des-acyl ghrelin is a peptide consisting of 28 amino acids but it differs from ghrelin in that the hydroxyl group at the side chain of the third serine residue from the amino terminus is not acylated by a fatty acid (n-octanoyl group) (SEQ ID NO:1).

Exemplary derivatives of des-acyl ghrelin include those compounds having the amino acid sequences of des-acyl ghrelin (for example, the amino acid sequences represented by SEQ ID NO:1 to SEQ ID NO:21), provided that one or several amino acids in the amino acid sequence are deleted, or substituted and/or added by other amino acids or non-amino acids, as well as compounds in which the amino terminus and/or carboxyl terminus of des-acyl ghrelin is modified (such as a compound amidated at the carboxyl terminus), etc. By choosing not to terminate the carboxyl terminus of an amino acid with a carboxylic acid but by amidating it to give a form that mimics a peptide bond, it becomes possible to find a minimum active unit of a shorter amino acid sequence. For example, any substances can be used if they have the amino acid sequence represented by SEQ ID NO:1, provided that one or several amino acids in the amino acid sequence starting from the 5^{th} up to the 28^{th} amino acid, preferably from the 11^{th} to the 28^{th} amino acid, more preferably from the 16^{th} to the 28^{th} amino acid, from the amino terminus are deleted, substituted and/or added, and if they act on a des-acyl ghrelin specific binding site.

To be more specific, peptides that retain a sequence of up to at least the 4^{th} from the amino terminus, say, up to the 5^{th} from the amino terminus, preferably up to the 7^{th} from the amino terminus, more preferably up to the 10^{th} from the amino terminus, and even more preferably up to the 15^{th} from the amino terminus of the amino acid sequences represented by SEQ ID NO:1 to SEQ ID NO:21 can advantageously be used as des-acyl ghrelin derivatives; for instance, one may advantageously use des-acyl ghrelin derivatives that have a sequence of up to at least the 5^{th}, preferably up to the 7^{th}, say, up to the 15^{th} from the amino terminus of des-acyl ghrelin, as exemplified by des-acyl ghrelin(1-5)-NH2 (SEQ ID NO:22), des-acyl ghrelin(1-7)-NH2(SEQ ID NO:23) and des-acyl ghrelin(1-15)NH2 (SEQ ID NO:24) that are described in Example 3.

Hereinafter, the number of amino acids as referred to in the passage reading that "one or several amino acids are substituted, deleted, inserted and/or added" is not particularly limited as long as the peptide comprising the amino acid sequence of interest or its derivatives have the desired function and it may range from about one to nine or from about one to four. If a large number of amino acids are substituted but by amino acids of similar properties (in electric charge and/or polarity), the desired function would not be lost.

It is desired that the amino acid sequences of peptides or their derivatives have homology of at least 70%, preferably at least 80%, more preferably at least 90%, even more preferably at least 95%, and most preferably at least 97%, in comparison with the natural type of amino acid sequence. This is also true with the des-acyl ghrelin derived from other animals (SEQ ID NO:2 to SEQ ID NO:21) and parts thereof (say, SEQ ID NO:22 to SEQ ID NO:24, preferably SEQ ID NO:23 and SEQ ID NO:24).

Other peptides or their derivatives can be designed by referring to the descriptions in documents such as the aforementioned Patent Document 1 concerning ghrelin. For example, preferred peptides or derivatives thereof include:
a peptide that has any one of the amino acid sequences represented by SEQ ID NO:1 to SEQ ID NO:21, preferably SEQ ID NO:1 to SEQ ID NO: 9, more preferably SEQ ID NO:1, or derivatives of the peptide;
a peptide that has any one of the amino acid sequences represented by SEQ ID NO:1 to SEQ ID NO:21, provided that one or several amino acids in the amino acid sequence starting from the 5^{th} amino acid from the amino terminus up to the carboxyl terminus, preferably starting from the 11^{th} amino acid from the amino terminus up to the carboxyl terminus, and more preferably starting from the 16^{th} amino acid from the amino acid terminus up to the carboxyl terminus, are deleted, substituted and/or added, or derivatives of the peptide;
a peptide having an amino acid sequence of up to at least the 4^{th} amino acid from the amino terminus, say, up to the 5^{th}, 7^{th}, 10^{th} or the 15^{th} amino acid from the amino terminus of the amino acid sequence represented by any one of SEQ ID NO:1 to SEQ ID NO:21, or derivatives of the peptide;
any one of the peptides described above or derivatives thereof, wherein the carboxyl terminus is an amide form;
any one of the peptides described above or derivatives thereof, wherein an acidic masking and a basic group are introduced at the carboxyl terminus;
any one of the peptides described above or derivatives thereof, wherein the amino acid at position 3 is a hydrophobic amino acid (e.g., an aromatic hydrophobic amino acid such as tryptophan, cyclohexylalanine or naphthylalanine, or an aliphatic hydrophobic amino acid such as leucine, isoleucine, ylleucine or valine);
any one of the peptides described above or derivatives thereof, wherein the amino acid at position 3 is basic;
any one of the peptides described above or derivatives thereof, wherein the amino acid at position 2 is an amino acid having a comparatively small side chain that will not constrain the degree of freedom of neighboring residues (as exemplified by serine, alanine or norvaline);
any one of the peptides described above or derivatives thereof, wherein the amino acids at positions 3 and 4 are both an L-form;
a peptide having 5 amino acid residues up to the 5^{th} amino acid from the amino terminus of the amino acid sequence represented by SEQ ID NO:1, with the 5^{th} amino acid being an amide form, or derivatives of the peptide;
a peptide having 7 amino acid residues up to the 7^{th} amino acid from the amino terminus of the amino acid sequence represented by SEQ ID NO:1, with the 7th amino acid being an amide form, or derivatives of the peptide; and
a peptide having 15 amino acid residues up to the 15^{th} amino acid from the amino terminus of the amino acid sequence represented by SEQ ID NO:1, with the 15^{th} amino acid being an amide form, or derivatives of the peptide.

Hereinafter, usability as the substance according to the present invention can be evaluated by using techniques known to skilled artisans to determine the identity of a substance that acts on a des-acyl ghrelin specific binding site, namely, the identity of a substance having binding activity for a des-acyl ghrelin specific binding site, or to determine the identity of "a substance having the action for proliferating spinal neuronal cells" (say, the identity of "a substance having the action for promoting the incorporation of uridine"), as will be described later in Examples 1 to 3.

For instance, the identity of a substance that acts on a des-acyl ghrelin specific binding site, namely, the identity of a substance having binding activity for a des-acyl ghrelin specific binding site can be determined, as will be described later in Example 1, by first preparing samples from a tissue of interest (e.g., membrane fractions prepared from an extracted spinal cord tissue), then incubating the sample in the presence of a test substance, with or without an added radioactive ligand such as [¹²⁵I]-labeled des-acyl ghrelin, measuring the radioactivity of the samples, and analyzing the values obtained. For analysis of the values, techniques known to skilled artisans may be employed, as exemplified by the method of Scatchard and a program such as GraphPAD Prism 4 program, which are applied to calculate the maximum number of binding sites (Bmax) and the dissociation constant (Kd). The des-acyl ghrelin specific binding sites are yet to be
characterized specifically but one place of their occurrence is in rat spinal cord cells, in particular, on rat spinal cord cell membranes.

Further, by performing incubation with the radioactive ligand added together with the test substance as noted above, the mode of binding (e.g., competitive, noncompetitive, uncompetitive, or mixed) can also be investigated. For example, the binding of a labeled des-acyl ghrelin is displaced by an unlabeled des-acyl ghrelin at concentrations near 10⁻³ M and above (IC₅₀ was 23.52 nM).

In addition, the identity of "a substance having the action for proliferating spinal neurons" (say, the identity of "a substance having the action for promoting the incorporation of uridine") can be examined by, for example, using bromodeoxyuridine (BrdU) which is an analogue of thymidine that can be incorporated into DNA specifically for the S phase of the cell cycle. For example, as will be described later in Examples 2 and 3, cells are cultured in a BrdU-supplemented medium in the presence or absence of the test substance so that BrdU is incorporated into the cells and, thereafter, an anti-BrdU antibody is used to stain those cells which have incorporated BrdU. In this case, double staining for both a marker for a specified cell (say, the neuronal precursor marker Nestin or the neuron marker Map2 (microtubule-associated protein 2)) and BrdU can also be performed.
Another method of investigation comprises culturing cells in a BrdU-supplemented medium in the presence or absence of the test substance so that they incorporate BrdU and, thereafter, measuring the amount of BrdU incorporation into the cells by the ELISA technique.

The criterion for judgment is as follows: if more of the cells cultured in the presence of the test substance are found to have incorporated stained BrdU than the cells (control) cultured in the absence of the test substance, the test substance of interest may be held as one that has the action for proliferating spinal neuronal cells. Any significant difference from the control suffices without particular limitation; however, the number of the cells that were cultured in the presence of the test substance and which incorporated the stained BrdU is preferably at least 105%, more preferably at least 110%, in comparison with the control.

If the cells cultured in the presence of the test substance are found to have incorporated more BrdU than the cells (control) cultured in the absence of the test substance, the test substance of interest may also be held as one that has the action for proliferating spinal neuronal cells. Any significant difference from the control suffices without particular limitation; however, the amount of BrdU that was incorporated into the cells cultured in the presence of the test substance is preferably at least 105%, more preferably at least 110%, in comparison with the control.

The substance thus confirmed to have the action for proliferating spinal neuronal cells may be administered, as will be typically described later in Example 4, into a rat model suffering from a damaged spinal cord in combination with the transplantation of spinal neuronal cells, whereupon it can be confirmed that the substance is useful for the recovery of an individual from the spinal cord injury.

The des-acyl ghrelin and its derivatives according to the present invention can be obtained by conventional procedures (see, for example, J. Med. Chem., 43, pp. 4370-4376, 2000). For instance, they can be isolated from naturally occurring raw materials or can be obtained by removing fatty acids from the ghrelin isolated from naturally occurring raw materials; alternatively, they can be produced by the recombinant DNA technology and/or chemical synthesis. For example, in a production process using the recombinant DNA technology, host cells transformed with an expression vector having DNA that encodes the peptide according to the present invention are cultured and the intended peptide is harvested from the culture, whereby the des-acyl ghrelin or its derivatives according to the present invention can also be obtained.

Vectors into which a gene is to be incorporated include *E. coli* vectors (e.g., pBR322, pUC18, and pUC19), *B. subtilis* vectors (e.g., pUB110, pTP5 and pC194), yeast vectors (e.g., YEp type, YRp type and YIp type), as well as animal cell vectors (e.g., retrovirus and vaccinia virus); any other vectors can be used as long as they can retain the intended gene stably within host cells. These vectors are introduced into suitable host cells. Methods that can be utilized to incorporate the intended gene into plasmids and to introduce them into host cells are exemplified by the methods described in Molecular Cloning (Sambrook et al., 1989).

In order to express the intended peptide gene in the above-mentioned plasmids, promoters are connected upstream of that gene in a functional manner.

Any promoters can be used in the present invention as long as they are appropriate for the host cell that is used to express the intended gene. For instance, if the host to be transformed is of the genus *Escherichia*, an lac promoter, a trp promoter, an lpp promoter, a λPL promoter, an recA promoter and the like can be used; in the case of the genus *Bacillus*, an SPO1 promoter, an SPO2 promoter and the like can be used; in the case of yeasts, a GAP promoter, a PHO5 promoter, an ADH promoter and the like can be used; in the case of animal cells, an SV40-derived promoter, a retrovirus-derived promoter and the like can be used.

The thus obtained vectors harboring the intended gene are used to transform the host cells. Exemplary host cells that can be used are bacteria (e.g., of the genus *Escherichia* and the genus Bacillus), yeasts (e.g., of the genus *Saccharomyces*, the genus Pichia, and the genus *Candida*), and animal cells (e.g., CHO cell and COS cell). Liquid media are suitable for use in culture and it is particularly preferred that they contain a carbon source, a nitrogen source and other nutrients that are necessary for the growth of the transformed cells that are to be cultured. If desired, vitamins, growth secretagogues, sera and the like may optionally be added.

After the cultivation, the substance according to the present invention is separated and purified from the culture by conventional procedures. For example, in order to extract the intended substance from the cultured fungus bodies or cells, the fungus bodies or cells are collected after the cultivation and then suspended in a buffer solution containing a protein denaturing agent (e.g., guanidine hydrochloride) and after they are sonicated or otherwise disrupted, the fungus bodies or cells are centrifuged. Subsequently, the intended substance is purified from the supernatant and this can be achieved by suitably combining separating and purifying methods such as gel filtration, ultrafiltration, dialysis, SDS-PAGE and various chromatographic techniques in consideration of the molecular weight of the intended substance, its solubility, electric charge (isoelectric point), affinity, and the like.

The substances that act on a des-acyl ghrelin specific binding site according to the present invention (e.g., des-acyl ghrelin and its derivatives) can be chemically synthesized by conventional techniques. For example, an amino acid having protective groups attached thereto is condensed by the liquid-phase method and/or the solid-phase method to extend the peptide chain and all protective groups are removed with an acid, the resulting crude product being then purified by the above-mentioned methods of purification to obtain the intended substance.

A variety of methods are already known for producing peptides and the peptides that are contained in the substances according to the present invention can also be produced easily in accordance with known methods; for example, classical methods of peptide synthesis may be complied with or easy production can also be achieved in accordance with the solid-phase method.

If desired, a method in which the recombinant DNA technology is combined with chemical synthesis may be employed to produce peptide compounds; a fragment containing modified amino acid residues is produced by chemical synthesis and another fragment that does not contain any modified amino acid residues is produced using the recombinant DNA technology, with the respective fragments being then fused (see, for example, Patent Document 1).

Preferred materials that can be used in the present invention as salts of the substances that act on a des-acyl ghrelin specific binding site (e.g., des-acyl ghrelin and its derivatives) are pharmaceutically acceptable salts and they include, for example, salts with inorganic bases, salts with organic bases, salts with inorganic acids, salts with organic acids, and salts with basic or acidic amino acids.

Advantageous examples of salts with inorganic bases include but are not limited to: alkali metal salts such as sodium salts and potassium salts; alkaline earth metal salts such as calcium salts and magnesium salts; as well as aluminum salts and ammonium salts.

Advantageous examples of salts with organic bases include, but are not limited to, salts with trimethylamine, triethylamine, pyridine, picoline, ethanolamine, diethanolamine, triethanolamine, dicyclohexylamine, and N,N'-dibenzylethylenediamine.

Advantageous examples of salts with inorganic acids include, but are not limited to, salts with hydrochloric acid, hydrobromic acid, nitric acid, sulfuric acid, and phosphoric acid.

Advantageous examples of salts with organic acids include, but are not limited to, salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Advantageous examples of salts with basic amino acids include, but are not limited to, salts with arginine, lysine, and ornithine, and advantageous examples of salts with acidic amino acids include, but are not limited to, salts with aspartic acid and glutamic acid.

Among the salts listed above, sodium salts and potassium salts in particular are most preferred.

Note that the cells which are allowed to proliferate by the substances that act on a des-acyl ghrelin specific binding site according to the present invention are positive (immunostained) for both Map2 and Nestin; based on this fact, the term "spinal neuronal cells" as used herein means spinal nerve cells or spinal neuronal precursor cells.

The proliferation of cells can be investigated by techniques known to skilled artisans; for example, it can be investigated by measuring the amount of incorporation of bromodeoxyuridine (BrdU) which is an analogue of thymidine that can be incorporated into DNA specifically for the S phase of the cell cycle or by counting the number of cells into which BrdU has been incorporated. Such measurement or counting may be performed using the methods described above or the methods that will be described later in the Examples.

The substances that act on a des-acyl ghrelin specific binding site according to the present invention (e.g., des-acyl ghrelin) have been found to possess the action for proliferating spinal neurons as will be shown specifically hereinafter in the Examples and it is particularly noted that they can achieve a significant increase in the number of spinal neuronal cells in embryos. Such cell proliferating action can occur with or without mediation by the des-acyl ghrelin specific binding site.

Thus, the substances that act on a des-acyl ghrelin specific binding site according to the present invention (e.g., des-acyl ghrelin) or pharmaceutically acceptable salts thereof can be used as an active ingredient in a spinal neuron damage treating agent for use in the treatment of spinal neuron damage, an agent for promoting the proliferation of spinal neuronal cells in the culture of spinal neurons, and an agent for promoting the regeneration of spinal nerves after transplantation of cultured spinal neuronal cells.

In addition, by administering the substances that act on a des-acyl ghrelin specific binding site according to the present invention (e.g., des-acyl ghrelin) or pharmaceutically acceptable salts thereof, they can be used in a method for treating spinal neuron damage in an individual, a method for promoting the proliferation of cultured spinal neuronal cells in the culture of spinal neuronal cells, and a method for promoting the regeneration of spinal nerves after transplantation of cultured spinal neuronal cells in an individual.

Furthermore, the substances that act on a des-acyl ghrelin specific binding site according to the present invention (e.g., des-acyl ghrelin) or pharmaceutically acceptable salts thereof can be used to produce a spinal neuron damage treating agent for use in the treatment of spinal neuron damage, an agent for promoting the proliferation of cultured spinal neuronal cells in the culture of spinal neurons, and an agent for promoting the regeneration of spinal nerves after transplantation of cultured spinal neuronal cells.

The drugs of the present invention that contain the substances that act on a des-acyl ghrelin specific binding site (e.g., des-acyl ghrelin) or pharmaceutically acceptable salts thereof as an active ingredient may be mixed with pharmacologically acceptable carriers, excipients, extenders and the like for use in individuals (e.g. human, mouse, rat, rabbit, canine, feline, bovine, equine, porcine, and monkey).

The drugs of the present invention (treating agent and promoting agent) are preferably administered to an individual under regenerative therapy for spinal nerves parenterally, as by intravenous, subcutaneous or intramuscular injection, in a single dose or divided portions of the required quantity; if the individual is a human adult and particularly in the case where he or she is under treatment at home, intranasal administration, pulmonary administration or suppository administration is desirable.

In the present invention, the dosage of the drug is not particularly limited and can be chosen as appropriate for various factors such as the purpose of its use, the age of the individual to which it is to be administered, their body weight, the kind of the individual, the severity of the disease, the state of nutrition, and the drug to be combined for use; if it is to be administered to a human adult in a single dose or divided portions, the substance that acts on a des-acyl ghrelin specific binding site (e.g., des-acyl ghrelin) or a salt thereof is preferably administered in amounts ranging from 0.001 mg to 100 mg, more desirably from 0.01 mg to 10 mg.

As for the period of administration, the above-mentioned dosage is preferably administered once to several times daily for 4 to 24 weeks, more preferably for 4 to 12 weeks.

Pharmaceutically acceptable carriers that can be used are a variety of organic or inorganic carrier substances that are commonly used as pharmaceutical necessities; they are incorporated as an excipient, a lubricant, a binder and a disintegrant in solid preparations or as a solvent, a solubilizing agent, a suspending agent, a tonicity agent, a buffer, a soothing agent, etc. in liquid preparations.

If necessary, pharmaceutical additives may be used, as exemplified by preservatives, antioxidants, coloring agents, and sweeteners.

Advantageous examples of excipients include lactose, sucrose, D-mannitol, starch, microcrystalline cellulose, light silicic anhydride, etc.

Advantageous examples of lubricants include magnesium stearate, calcium stearate, talc, colloidal silica, etc.

Advantageous examples of binders include microcrystalline cellulose, sucrose, D-mannitol, dextrin, hydroxypropyl cellulose, hydroxypropyl methylcellulose, polyvinylpyrrolidone, etc.

Advantageous examples of disintegrants include starch, carboxymethylcellulose, carboxymethylcellulose calcium, croscarmelose sodium, carboxymethylstarch sodium, etc.

Advantageous examples of solvents include water for injection, alcohols, propylene glycol, macrogol, sesame oil, corn oil, etc.

Advantageous examples of solubilizing agents include polyethylene glycol, propylene glycol, D-mannitol, benzyl benzoate, ethanol, trisaminomethane, cholesterol, triethanolamine, sodium carbonate, sodium citrate, etc.

Advantageous examples of suspending agents include: surface active agents such as stearyl triethanolamine, sodium lauryl sulfate, lauryl aminopropionic acid, lecithin, benzalkonium chloride, benzethonium chloride, glyceryl monostearate, etc.; and hydrophilic polymers such as polyvinyl alcohol, polyvinylpyrrolidone, carboxymethylcellulose sodium, methylcellulose, hydroxymethyl cellulose, hydroxyethyl cellulose, hydroxypropyl cellulose, etc.

Advantageous examples of tonicity agents include sodium chloride, glycerin, D-mannitol, etc.

Advantageous examples of buffers include buffer solutions of phosphates, acetates, carbonates, citrates, etc.

Advantageous examples of soothing agents include benzyl alcohol, etc.

Advantageous examples of preservatives include paraoxybenzoic acid esters, chlorobutanol, benzyl alcohol, phenethyl alcohol, dehydroacetic acid, sorbic acid, etc.

Advantageous examples of antioxidants include sulfurous acid salts, ascorbic acid, etc.

The pharmaceutical preparations of the present invention preferably assume dosage forms that are suitable for parenteral administration; dosage forms suitable for parenteral administration include, for example, injections for intravenous, intracutaneous, subcutaneous, intramuscular or other administration, as well as drops, suppositories, transdermal drug delivery systems, transmucosal drug delivery systems, and inhalants; the above-mentioned injections are a preferred dosage form and particularly in the case where the individual is a human adult who is under treatment at home, transmucosal drug delivery systems, inhalants, suppositories and the like are also preferred as dosage forms. These dosage forms are variously known to skilled artisans, who may appropriately select dosage forms that are suitable for the desired route of administration and, if necessary, may use one or two or more pharmaceutical additives available in the art to produce preparations in the form of a pharmaceutical composition.

For example, pharmaceuticals in the form of an injection or a drop can be prepared and provided by the following procedure: the active ingredient, or the substance that acts on a des-acyl ghrelin specific binding site (e.g., des-acyl ghrelin), is dissolved in distilled water for injection together with one or two or more suitable pharmaceutical additives such as a buffer solution, a sugar solution, a tonicity agent, a pH modifier, a soothing agent and a preservative, subjecting the solution to sterilizing filtration (by passage through a filter), and filling ampoules or vials with the sterilized solution; alternatively, the solution subjected to sterilizing filtration is freeze-dried to formulate a lyophilized preparation. Exemplary additives that can be used include: sugars such as glucose, mannitol, xylitol and lactose; hydrophilic polymers such as polyethylene glycol; alcohols such as glycerol; amino acids such as glycine; proteins such as serum albumin; salts such as NaCl and sodium citrate; acids such as acetic acid, tartaric acid, and ascorbic acid; surface active agents such as Tween 80; and reducing agents such as sodium sulfite. These preparations can be used as an injection or a drop that is prepared by dissolving them in distilled water for injection, physiological saline or the like that are added just before use. For transmucosal administration, intranasal delivery systems (transnasal delivery systems) such as a nasal drop and an intranasal spray are also advantageous, and for transpulmonary administration, an inhalant or the like is also advantageous.

The content of the substance that acts on a des-acyl ghrelin specific binding site (e.g., des-acyl ghrelin) or a salt thereof in one preparation ranges from 0.001 mg to 100 mg, preferably from 0.01 mg to 10 mg, and the frequency of administration is desirably from once to several times a day.

In one method of treating the isolated spinal neuronal cells, they are first incubated in a culture broth and to the incubating broth, the substance that acts on a desacyl ghrelin specific binding site (e.g., des-acyl ghrelin), as prepared by sterilizing filtration or autoclaving sterilization is added in an amount ranging from 0.1 nM to 1 µM, preferably from 1 nM to 100 nM. The treatment can also be realized by adding 0.0000001 mg/L to 0.1 mg/L of the substance that acts on a des-acyl ghrelin specific binding site (e.g., des-acyl ghrelin) or a salt thereof. As will be shown in Examples 2 and 3, this treatment enables promoting the proliferation of spinal neuronal cells that are inherently very slow to proliferate.

Furthermore, as will be described in Example 4, it becomes possible to promote the recovery of an individual from dysfunction due to spinal cord injury.

Hereinafter, any reference to the quantity that is made about the substance (e.g., a peptide) or its salt (for example, "containing 0.001 mg to 100 mg of the substance or a pharmaceutically acceptable salt thereof" or "the content of the substance or its salt ranges from 0.0000001 mg/L to 0.1 mg/L") indicates, unless otherwise noted, the amount of that substance itself (e.g., a peptide). In other words, unless noted otherwise, the quantity of the salt is indicated as an equivalent amount of the corresponding substance (e.g., a peptide).

### EXAMPLES

On the following pages, the present invention is shown specifically by reference to Examples.

### Example 1: Des-acyl Specific Binding in Rat Embryos

With [¹²⁵I]-labelled des-acyl ghrelin used as a radioactive ligand, the binding of des-acyl ghrelin to the tissue membranes of fetal spinal cord was studied. From the embryos of pregnant Wistar rats at days 13 to 19, spinal cord tissues were extracted to prepare membrane fractions (30,000 x g pellet). Membrane fractions whose protein content was found to be 10 µg by the Lowry method were incubated at 4 °C for an hour in a [¹²⁵I]-labelled des-acyl ghrelin assay buffer (50 mM Tris-HCl, 2.5 mM EGTA, 0.1% BSA, protease inhibitor cocktail (Sigma, MO, USA), pH 7.4) (final volume: 0.5 ml) with the concentration increasing from 0.13 to 16.64 nM. Parallel incubations in the presence of 1.0 µM unlabelled des-acyl ghrelin were conducted to determine non-specific binding, which was subtracted from total binding to obtain specific binding values. For competition assay, the tissue membranes were incubated with 0.1 nM labeled des-acyl ghrelin and either unlabeled des-acyl ghrelin or ghrelin at 4 °C for an hour. Thereafter, the reaction solution was filtered through Whatman GF/B filters to separate the membrane-binding fractions. The radioactivity of the membranes on the filters was measured with a gamma counter. The method of Scatchard and the GraphPAD Prism 4 program (GraphPAD Software, CA, USA) were used to calculate the maximum number of binding sites (Bmax) and the dissociation constant (Kd).

The results are shown in FIG. 1. For the labeled des-acyl ghrelin, specific, high-affinity and saturable binding was observed (Kd = 3.467; Bmax = 8.79 fmol/mg protein). The binding of the labeled des-acyl ghrelin to the membrane fractions of spinal cord was displaced by the unlabeled des-acyl ghrelin at concentrations near 10⁻³ M and above. The IC₅₀ of the des-acyl ghrelin was 23.52 nM.

From the foregoing, the expression of des-acyl ghrelin specific binding sites in the membrane fractions of fetal spinal cord was verified.

### Example 2: Des-acyl Ghrelin's Action for Promoting BrdU Incorporation into Cultured Rat Fetal Spinal Neurons and for Proliferating Cells

(1) Embryos were extracted from a pregnant Wistar rat at day 17 under anesthesia. Spinal cords were collected from these embryos and subjected to papain digestion in Hank's balanced salt solution; as a result of subsequent mechanical separation by pipetting, a dispersion of fetal spinal neurons was obtained. The thus obtained cells were Map2- and Nestin-positive and, hence, they proved to be spinal nerve cells and spinal neuronal precursor cells. These dispersed cells were suspended in a DMEM medium containing NaHCO₃, 5% fetal bovine serum, penicillin (100 U/mL) and streptomycin (100 µg/mL), followed by plating onto laminin-coated 96-well multi-plates at 10⁵ cells per well. After incubation for 4 days, des-acyl ghrelin (0.03-300 nM) was added to the culture and incubation was conducted for 12 hours; then, 5-bromo-2'-deoxyuridine (BrdU) (10 nM) was added and incubation was conducted for an additional 6 hours; then, the des-acyl ghrelin was investigated for its action on BrdU incorporation.

(2) After the end of the above incubation, the cells were recovered and using Cell Proliferation ELISA Kit (Roche Diagnostic GmbH, Mannheim, Germany), the amount of BrdU incorporation into the fetal spinal neuronal cells was measured to investigate the action for proliferating the spinal neuronal cells.

The results are shown in FIG. 2A. When ghrelin was allowed to act on the cultured fetal spinal neuronal cells, 3 nM and more of des-acyl ghrelin could increase the BrdU incorporation into the cells.

(3) After the end of the above incubation (1), the cells were fixed with methanol and glacial acetic acid, had their DNA denatured with 2 N HCl; thereafter, the BrdU incorporated into the cells was measured by detecting the BrdU positive cells using a rat anti-BrdU monoclonal antibody (1:1000, Abcam, Cambridge, UK) as a primary antibody, and also using Cy^{™} 3-conjugated donkey anti-rat IgG polyclonal antibody (1:1000, Jackson ImmunoResearch Laboratories, Inc., PA, USA) as a secondary antibody, in order to investigate the action for proliferating spinal cord cells. Comparison was made with a control in which physiological saline was allowed to act instead of des-acyl ghrelin.

The results are shown in FIGS. 2B and 2C. Compared to the case where physiological saline rather than des-acyl ghrelin was allowed to act (FIG. 2B), the BrdU incorporation increased and so did the number of cells that incorporated BrdU when des-acyl ghrelin was allowed to act (FIG. 2C).
Thus, the action of des-acyl ghrelin for proliferating spinal neuronal cells was also confirmed at the cellular level.

As shown above, when des-acyl ghrelin was allowed to act on the cultured fetal spinal neuronal cells, the BrdU incorporation increased and, at the same time, the number of cells that incorporated BrdU increased; thus, it became clear that des-acyl ghrelin has the action of proliferating spinal neuronal cells.

### Example 3: Action of Des-acyl Ghrelin and Derivatives Thereof for Promoting BrdU Incorporation into Cultured Spinal Neuronal Cells

Embryos were extracted from a pregnant Wistar rat at day 16 by opening the abdomen under anesthesia. Spinal cords were collected from these embryos and subjected to papain digestion in Hank's balanced salt solution; as a result of subsequent mechanical separation by pipetting, a dispersion of fetal spinal nerve cells was obtained. After being filtered and centrifuged, the dispersed cells were suspended in a DMEM medium containing NaHCO₃, 5% fetal bovine serum, penicillin (100 U/mL) and streptomycin (100 µg/mL), followed by plating onto laminin-coated 96-well multi-plates at 10⁵ cells per well. After incubation for 4 days, 5-bromo-2'-deoxyuridine (BrdU) (10 µM) was added and incubation was conducted for 6 hours; further, rat des-acyl ghrelin, des-acyl ghrelin(1-5)-NH2 (GSSFL-NH2, SEQ ID NO:22), des-acyl ghrelin(1-7)-NH2 (GSSFLSP-NH2, SEQ ID NO:23) or des-acyl ghrelin(1-15)-NH2 (GSSFLSPEHQRVQQR-NH2, SEQ ID NO:24) was added in amounts of 0.03 - 3 nM and incubation was conducted for 12 hours before investigating the action of des-acyl ghrelin and its derivatives on BrdU incorporation.

After the end of incubation, the cells were recovered and using Cell Proliferation ELISA Kit (Roche Diagnostic GmbH, Mannheim, Germany), the amount of BrdU incorporation into the cells was measured to investigate the action for proliferating the spinal neuronal cells.

The results are shown in Table 1. When des-acyl ghrelin was allowed to act on spinal neuronal cells, the BrdU incorporation into the cells was increased by 0.03 to 3 nM or more of des-acyl ghrelin, thus confirming that des-acyl ghrelin has the action of proliferating spinal neuronal cells.

This action for proliferating spinal neuronal cells was also recognized when the derivatives of des-acyl ghrelin were used and particularly in the case where des-acyl ghrelin(1-7)-NH2 or des-acyl ghrelin(1-15)-NH2 was allowed to act, the BrdU incorporation into the cells increased markedly, whereby it was confirmed at the cellular level that des-acyl ghrelin, as well as its derivatives that had peptide chains starting from at least the 5^{th}, preferably from at least the 7^{th}, amino acid from the amino terminus of des-acyl ghrelin have the action for increasing the number of spinal neurons. cells, as do ghrelin or derivatives thereof.

**[Table 1]**

| Action of Des-acyl Ghrelin and Derivatives Thereof for Proliferating Spinal Neurons | | | | | | |
|---|---|---|---|---|---|---|
| Des-acyl ghrelin derivative | Concentration (nM) | n | Mean | ± | S.D. | t-test |
| Des-acyl ghrelin | 0.03 | 6 | 113.6 | ± | 15.8 | NSD |
| | 0.3 | 6 | 112.6 | ± | 6.8 | p <0.01 |
| | 3 | 6 | 108.2 | ± | 6.4 | p <0.05 |
| | | | | | | |
| Desacyl-ghrelin (1-5)-NH ₂ | 0.03 | 6 | 105.8 | ± | 10.2 | NSD |
| | 0.3 | 6 | 110.6 | ± | 12.3 | NSD |
| | 3 | 6 | 113.5 | ± | 20.4 | NSD |
| | | | | | | |
| Desacyl-ghrelin (1-7)-NH ₂ | 0.03 | 6 | 106.8 | ± | 12.0 | NSD |
| | 0.3 | 6 | 111.1 | ± | 11.1 | p <0.05 |
| | 3 | 6 | 106.4 | ± | 6.7 | NSD |
| | | | | | | |
| Desacyl-ghrelin (1-15)-NH ₂ | 0.03 | 6 | 97.6 | ± | 17.4 | NSD |
| | 0.3 | 6 | 113.2 | ± | 16.2 | NSD |
| | 3 | 6 | 117.6 | ± | 12.0 | p <0.01 |
| | | | | | | |
| Control | 0 | 12 | 100.0 | ± | 6.1 | - |

| | | | | | | |
|---|---|---|---|---|---|---|
| NSD: No significant difference | | | | | | |

### Example 4: Assessment of Grafting Spinal Neurons and Locally Administering Des-acyl Ghrelin to Rat Models with Injured Spinal Cord

In accordance with the procedure described in Morino, T. et al, Neuroscience Research, 2003, vol. 46, pp 309-318, the grafting of spinal neuronal cells and local administration of des-acyl ghrelin to rat models with injured spinal cord were assessed. Male SD rats (8 to 9 weeks old) were anesthetized with pentobarbital and incised in the back to expose a vertebra (L11). After excising the vertebral arch, a dental drill was used to open a window with a diameter of about 3 cm, and a stainless steel needle with a silicone rubber fixed thereto was applied vertically, followed by loading of the top with a 20-g weight. Following a compression time of 30 minutes, a damaging drop equivalent to a weight of about 100 g was finally applied from above to construct models suffering a movement disorder in the lower limbs. The animals were divided into three groups: 1) a group under sham operation; 2) a control group with injured spinal cord; and 3) a group with injured spinal cord, treated by cell grafting, and locally administered with des-acyl ghrelin. The group under sham operation received laminectomy but was simply sutured in the muscle and skin thereafter. After injuring the spinal cord, cultured spinal neuronal cells (10⁵ cells/25 µL) were grafted subdurally. Rat des-acyl ghrelin was administered subdurally in an amount of 1 nmol together with the cells.

Twenty-four hours after the operation, the rats were transferred into a see-through cage and observed for the frequency of their standing up (by 3-minute observation). The principal item of observation was the frequency per unit time of the rat standing up (in such a posture that it lifted the upper limbs and supported the body weight only with the lower limbs) was counted to assess the function of its hind limbs. The results are shown in Table 2.

**[Table 2]**

| Assessment of Grafting Spinal Neurons and Locally Administering Des-acyl Ghrelin to Rat Models with Injured Spinal Cord | | | | |
|---|---|---|---|---|
| Group | Initial | 24 hours after operation | t-test* | t-test** |
| Group under sham operation | 10.7 ± 3.8 | 4.3 ± 4.2 | - | - |
| Control group with injured spinal cord | 10.7 ± 2.5 | 1.3 ± 0.6 | NS | - |
| Group with injured spinal cord, grafted with cells, and administered with des-acyl ghrelin | 15.0 ± 2.0 | 8.0 ± 0.0 | NS | p < 0.01 |

| | | | | |
|---|---|---|---|---|
| The numerals indicate the frequency of a rat standing up per unit time. Note*: Group under sham operation (initial) vs group with injured spinal cord (initial) Note**: Control group with injured spinal cord (after operation) vs group with injured spinal cord and treated (after operation) NS: Not significant | | | | |

As Table 2 shows, the frequency of standing up in the control group for models with injured spinal cord decreased from the initial 10.7 times to 1.3 times; however, the combination of cell grafting and local administration of des-acyl ghrelin led to a recovery in the frequency of standing up.

## Claims

1. A spinal neuron damage treating agent containing a substance that acts on a des-acyl ghrelin specific binding site or a pharmaceutically acceptable salt thereof as an active ingredient.

2. The treating agent of claim 1, wherein the substance is a peptide selected from the group consisting of:
(1) a peptide having the amino acid sequence represented by any one of SEQ ID NO:1 to SEQ ID NO:21;
(2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
(3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.

3. The treating agent of claim 2, wherein the substance is a peptide having the amino acid sequence represented by SEQ ID NO:1.

4. The treating agent as recited in any one of claims 1 to 3, which contains 0.001 mg to 100 mg of the substance or pharmaceutically acceptable salt thereof.

5. An agent for promoting the proliferation of spinal neuronal cells in the culture of spinal neuronal cells that contains a substance that acts on a des-acyl ghrelin specific binding site or a salt thereof as an active ingredient.

6. The promoting agent of claim 5, wherein the substance is a peptide selected from the group consisting of:
(1) a peptide having the amino acid sequence represented by any one of SEQ ID NO:1 to SEQ ID NO:21;
(2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
(3) a peptide having a sequence of up to at least the 4^{th} amino acid from the amino terminus of the amino acid sequence represented by any one of SEQ ID NO:1 to SEQ ID NO:21 and having a spinal neuronal cells proliferating action,
or a derivative thereof.

7. The promoting agent of claim 6, wherein the substance is a peptide having the amino acid sequence represented by SEQ ID NO:1.

8. The promoting agent as recited in any one of claims 5 to 7, wherein the content of the substance or salt thereof in the culture medium for spinal neuronal cells is from 0.0000001 mg/L to 0.1 mg/L.

9. An agent for promoting the regeneration of spinal nerves after transplantation of cultured spinal neuronal cells that contains a substance that acts on a des-acyl ghrelin specific binding site or a pharmaceutically acceptable salt thereof as an active ingredient.

10. The promoting agent of claim 9, wherein the substance is a peptide selected from the group consisting of:
(1) a peptide having the amino acid sequence represented by any one of SEQ ID NO:1 to SEQ ID NO:21;
(2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
(3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.

11. The promoting agent of claim 10, wherein the substance is a peptide having the amino acid sequence represented by SEQ ID NO:1.

12. The promoting agent as recited in any one of claims 9 to 11, which contains 0.001 mg to 100 mg of the substance or pharmaceutically acceptable salt thereof.

13. A method for promoting the proliferation of cultured spinal neuronal cells, **characterized by** using a substance that acts on a des-acyl ghrelin specific binding site or a salt thereof.

14. The method of claim 13, wherein the substance is a peptide selected from the group consisting of:
(1) a peptide having the amino acid sequence represented by any one of SEQ ID NO:1 to SEQ ID NO:21;
(2) a peptide having a sequence of up to the 4^{th} amino acid from the amino terminus of the amino acid sequence represented by any one of SEQ ID NO:1 to SEQ ID NO:21 and wherein one or several amino acids in the amino acid sequence of from the 5^{th} amino acid from the amino terminus up to the carboxyl terminus are deleted, substituted and/or added, the peptide having a spinal neuronal cells proliferating action; and
(3) a peptide having a sequence of up to at least the 4^{th} amino acid from the amino terminus of the amino acid sequence represented by any one of SEQ ID NO:1 to SEQ ID NO:21 and having a spinal neuronal cells proliferating action, or a derivative thereof.

15. The method of claim 14, wherein the substance is a peptide having the amino acid sequence represented by SEQ ID NO:1.

16. The method as recited in any one of claims 13 to 15, wherein the content of the substance or salt thereof in the culture medium for the cultured spinal neuronal cells is from 0.0000001 mg/L to 0.1 mg/L.


<110> University of Miyazaki,
Asubio Pharma.Co.Ltd.,
Japan as represented by President of National Cardiovascular Center
<120> SPINAL NERVE REPAIR PROMOTING THERAPEUTICS CONTAINING DES-ACYL GHRELIN OR ITS DERIVATIVES AS AN
ACTIVE INGREDIENT
<130> FA0011-07113
<150> JP 2006-220695
<151> 2006-8-11
<160> 24

<210> 1
<211> 28
<212> PRT
<213> Homo sapiens
<221> PEPTIDE
<223> Amino acid sequence for human endogenous peptides of growth hormo
ne secretagogue
<400> 1
Gly Ser Ser Phe Leu Ser Pro Glu His Gln Arg Val Gln Gln Arg Lys
1               5                   10                  15
Glu Ser Lys Lys Pro Pro Ala Lys Leu Gln Pro Arg
            20                  25

<210> 2
<211> 27
<212> PRT
<213> Homo sapiens
<221> PEPTIDE
<223> Amino acid sequence for human endogenous peptides (27 amino acids
) of growth hormone secretagogue
<400> 2
Gly Ser Ser Phe Leu Ser Pro Glu His Gln Arg Val Gln Arg Lys Glu
1               5                   10                  15
Ser Lys Lys Pro Pro Ala Lys Leu Gln Pro Arg
            20                  25

<210> 3
<211> 28
<212> PRT
<213> Rattus norvegicus
<221> PEPTIDE
<223> Amino acid sequence for rat endogenous peptides of growth hormone
secretagogue
<400> 3
Gly Ser Ser Phe Leu Ser Pro Glu His Gln Lys Ala Gln Gln Arg Lys
1               5                   10                  15
Glu Ser Lys Lys Pro Pro Ala Lys Leu Gln Pro Arg
            20                  25

<210> 4
<211> 27
<212> PRT
<213> Rattus norvegicus
<221> PEPTIDE
<223> Amiino acid sequence for rat endogenous peptides of growth hormon
e secretagogue
<400> 4
Gly Ser Ser Phe Leu Ser Pro Glu His Gln Lys Ala Gln Arg Lys Glu
1               5                   10                  15
Ser Lys Lys Pro Pro Ala Lys Leu Gln Pro Arg
            20                  25

<210> 5
<211> 28
<212> PRT
<213> Mus musculus
<221> PEPTIDE
<223> Amino acid sequence for mouse endogenous peptides of growth hormo
ne secretagogue
<400> 5
Gly Ser Ser Phe Leu Ser Pro Glu His Gln Lys Ala Gln Gln Arg Lys
1               5                   10                  15
Glu Ser Lys Lys Pro Pro Ala Lys Leu Gln Pro Arg
            20                  25

<210> 6

<211>  28
<212>  PRT
<213>  Sus scrofa
<221>  PEPTIDE
<223>  Amino acid sequence for porcine endogenous peptides of growth hor
       mone secretagogue
<400>  6
Gly Ser Ser Phe Leu Ser Pro Glu His Gln Lys Val Gln Gln Arg Lys
1               5                   10                  15
Glu Ser Lys Lys Pro Ala Ala Lys Leu Lys Pro Arg
            20                  25

<210>  7
<211>  27
<212>  PRT
<213>  Bos taurus
<221>  PEPTIDE
<223>  Amino acid sequence for bovine endogenous peptides (27 amino acid
       s) of growth hormone secretagogue
<400>  7
Gly Ser Ser Phe Leu Ser Pro Glu His Gln Lys Leu Gln Arg Lys Glu
1               5                   10                  15
Ala Lys Lys Pro Ser Gly Arg Leu Lys Pro Arg
            20                  25

<210>  8
<211>  27
<212>  PRT
<213>  Ovis aries
<221>  PEPTIDE
<223>  Amino acid sequence for ovine endogenous peptides (27 amino acids
       ) of growth hormone secretagogue
<400>  8
Gly Ser Ser Phe Leu Ser Pro Glu His Gln Lys Leu Gln Arg Lys Glu
1               5                   10                  15
Pro Lys Lys Pro Ser Gly Arg Leu Lys Pro Arg
            20                  25

<210>  9
<211>  28
<212>  PRT
<213>  Canis familiaris
<221>  PEPTIDE
<223>  Amino acid sequence for dog endogenous peptides of growth hormone
        secretagogue
<400>  9
Gly Ser Ser Phe Leu Ser Pro Glu His Gln Lys Leu Gln Gln Arg Lys
1               5                   10                  15
Glu Ser Lys Lys Pro Pro Ala Lys Leu Gln Pro Arg
            20                  25

<210>  10
<211>  21
<212>  PRT
<213>  Anguilla japonica
<221>  PEPTIDE
<223>  Amino acid sequence for eel endogenous peptides of growth hormone
        secretagogue. This peptide is amidated at C-terminus.
<400>  10
Gly Ser Ser Phe Leu Ser Pro Ser Gln Arg Pro Gln Gly Lys Asp Lys
1               5                   10                  15
Lys Pro Pro Arg Val
            20

<210>  11
<211>  23
<212>  PRT
<213>  Oncorhynchus mykiss
<221>  PEPTIDE
<223>  Amino acid sequence for rainbow trout endogenous peptides (23 ami
       no acids) of growth hormone secretagogue. This peptide is amidate
       d at C-terminus.
<400>  11
Gly Ser Ser Phe Leu Ser Pro Ser Gln Lys Pro Gln Val Arg Gln Gly
1               5                   10                  15
Lys Gly Lys Pro Pro Arg Val

            20

<210> 12
<211> 20
<212> PRT
<213> Oncorhynchus mykiss
<221> PEPTIDE
<223> Amino acid sequence for rainbow trout endogenous peptides (20 ami
no acids) of growth hormone secretagogue. This peptide is amidat
ed at C-terminus.
<400> 12
Gly Ser Ser Phe Leu Ser Pro Ser Gln Lys Pro Gln Gly Lys Gly Lys
1               5                   10                  15
Pro Pro Arg Val
            20

<210> 13
<211> 24
<212> PRT
<213> Gallus domesticus
<221> PEPTIDE
<223> Amino acid sequence for chicken endogenous peptides of growth hor
mone secretagogue
<400> 13

Gly Ser Ser Phe Leu Ser Pro Thr Tyr Lys Asn Ile Gln Gln Gln Lys
1               5                   10                  15
Gly Thr Arg Lys Pro Thr Ala Arg
            20

<210> 14
<211> 24
<212> PRT
<213> Gallus domesticus
<221> PEPTIDE
<223> Amino acid sequence for chicken endogenous peptides of growth hor
mone secretagogue
<400> 14
Gly Ser Ser Phe Leu Ser Pro Thr Tyr Lys Asn Ile Gln Gln Gln Lys
1               5                   10                  15
Asp Thr Arg Lys Pro Thr Ala Arg
            20

<210> 15
<211> 26
<212> PRT
<213> Gallus domesticus
<221> PEPTIDE
<223> Amino acid sequence for chicken endogenous peptides of growth hor
mone secretagogue
<400> 15
Gly Ser Ser Phe Leu Ser Pro Thr Tyr Lys Asn Ile Gln Gln Gln Lys
1               5                   10                  15
Asp Thr Arg Lys Pro Thr Ala Arg Leu His
            20                  25

<210> 16
<211> 27
<212> PRT
<213> Rana catesbeiana
<221> PEPTIDE
<223> Amino acid sequence for frog endogenous peptides of growth hormon
e secretagogue
<400> 16
Gly Leu Thr Phe Leu Ser Pro Ala Asp Met Gln Lys Ile Ala Glu Arg
1               5                   10                  15
Gln Ser Gln Asn Lys Leu Arg His Gly Asn Met
            20                  25

<210> 17
<211> 28
<212> PRT
<213> Rana catesbeiana
<221> PEPTIDE
<223> Amino acid sequence for frog endogenous peptides of growth hormon
e secretagogue

<400>  17
Gly Leu Thr Phe Leu Ser Pro Ala Asp Met Gln Lys Ile Ala Glu Arg
1               5                   10                  15
Gln Ser Gln Asn Lys Leu Arg His Gly Asn Met Asn
            20                  25

<210>  18
<211>  20
<212>  PRT
<213>  Tilapia nilotica
<221>  PEPTIDE
<223>  Amino acid sequence for tilapia endogenous peptides of growth hor
       mone secretagogue.  This peptide is amidated at C-terminus.
<400>  18
Gly Ser Ser Phe Leu Ser Pro Ser Gln Lys Pro Gln Asn Lys Val Lys
1               5                   10                  15
Ser Ser Arg Ile
            20

<210>  19
<211>  22
<212>  PRT
<213>  Silurus asotus
<221>  PEPTIDE
<223>  Amino acid sequence for catfish endogenous peptides of growth hor
       mone secretagogue.  This peptide is amidated at C-terminus.
<400>  19
Gly Ser Ser Phe Leu Ser Pro Thr Gln Lys Pro Gln Asn Arg Gly Asp
1               5                   10                  15
Arg Lys Pro Pro Arg Val
            20

<210>  20
<211>  23
<212>  PRT
<213>  Silurus asotus
<221>  PEPTIDE
<223>  Amino acid sequence for catfish endogenous peptides of growth hor
       mone secretagogue
<400>  20
Gly Ser Ser Phe Leu Ser Pro Thr Gln Lys Pro Gln Asn Arg Gly Asp
1               5                   10                  15
Arg Lys Pro Pro Arg Val Gly
            20

<210>  21
<211>  28
<212>  PRT
<213>  Equus caballus
<221>  PEPTIDE
<223>  Amino acid sequence for equine endogenous peptides of growth horm
       one secretagogue
<400>  21
Gly Ser Ser Phe Leu Ser Pro Glu His His Lys Val Gln His Arg Lys
1               5                   10                  15
Glu Ser Lys Lys Pro Pro Ala Lys Leu Lys Pro Arg
            20                  25

<210>  22
<211>  5
<212>  PRT
<213>  Homo sapiens
<221>  PEPTIDE
<223>  This peptide is amidated at C-terminus.
<400>  22
Gly Ser Ser Phe Leu
1               5

<210>  23
<211>  7
<212>  PRT
<213>  Homo sapiens
<221>  PEPTIDE
<223>  This peptide is amidated at C-terminus.
<400>  7
Gly Ser Ser Phe Leu Ser Pro

1                    5

<210>  24
<211>  15
<212>  PRT
<213>  Homo sapiens
<221>  PEPTIDE
<223>  This peptide is amidated at C-terminus.
<400>  15
Gly Ser Ser Phe Leu Ser Pro Glu His Gln Arg Val Gln Gln Arg
1               5                   10                  15